# EUROPEAN PATENT APPLICATION

(11) **EP 3 292 822 A2**
(43) Date of publication of application: **14.03.2018**
(21) Application number: 17180682.1
(22) Date of filing: 11.07.2017
(51) Int. Cl.: A61B 10/02, A61B 5/00

(54) **BIOPSY DEVICE**

(30) Priority: 15.08.2016 US 201662375028 P
(71) Applicant: Dune Medical Devices Ltd., 3088900 Caesarea (IL)
(72) Inventor: HASHIMSHONY, Dan, 3713227 Pardes Hana (IL); COHEN, Gil, 9782224 Jerusalem (IL); MANN, Chen, 1910000 Kibbutz Merchavia (IL); AHARONOWITZ, Gal, Sde Warburg 44935 (IL); GELTNER, Iddo, 40500 Even Yehuda (IL)
(74) Representative: Becker Kurig Straus

(57) **Abstract**

Biopsy devices are presented comprising: an inner needle comprising a tip for piercing tissue, and a cavity for receiving a tissue portion; an outer sheath moving with respect to the inner needle between a forward position of the sheath in which it totally covers the cavity and a backward position in which the cavity is totally uncovered, the outer sheath comprising a cutting edge at its front end configured to cut the tissue portion into the cavity while moving in a forward direction; and at least one of: a sensing unit mounted on the outer sheath such that the sensing unit is aligned with the cavity when the sheath is in the forward position to thereby sense tissue signals from the tissue portion; or a suction system for applying suction force in the cavity to pull the tissue portion into the cavity while the sheath moves in a backward direction.

## Description

### TECHNOLOGICAL FIELD

The present invention relates to surgical medical devices, and in particular devices for obtaining a tissue sample in vivo.

### BACKGROUND

In core biopsy procedures, the biopsy device typically consists of an inner needle, or a pointed tip stylet, which includes a sampling cavity close to the needle tip in the form of a trough, or a shallow receptacle, covered by an outer sheath, cannula or sleeve with a cutting edge and attached to a spring-loaded mechanism.

During a biopsy procedure, the biopsy device is inserted into the body, either in its closed state, i.e. the sheath is in a forward position covering the sampling cavity, or its open state, i.e. the sheath is in a retracted position revealing the sampling cavity. In the case the biopsy device is inserted into the body in its closed state, when approaching the biopsy sampling location, the inner needle is fired forwardly inside the body towards the biopsy sampling location to reveal the sampling cavity. The outer sheath, being provided with cutting means, is then moved forwardly to cut the tissue that fills the needle trough and keeps it in the needle trough.

Alternatively, in the case the biopsy device is inserted into the body in its open state, once the biopsy device is at the biopsy sampling location, only a forward movement of the outer sheath is performed, to cut tissue and enclose it in the sampling cavity.

Instead of or in addition to externally applied diagnostic imaging systems, such as x-ray or Ultrasound, aimed at guiding the device inside the body or locating tissue of interest to be sampled, some biopsy devices include sensor(s) aimed at characterizing the tissue in vivo, so to improve the localization of tissue of interest and the overall sampling procedure.

WO2011016034, assigned to the assignee of the present invention, discloses a surgical tool for use in a tissue removal procedure from a subject. The surgical tool has proximal and distal regions and at least one sensor for sensing one or more predetermined conditions located at a distal region of the surgical tool.

WO2009010960, assigned to the assignee of the present invention, discloses a medical device for use in tissue characterization and treatment. The device comprises a tissue characterization probe comprising an elongated carrier for carrying an array of tissue characterization sensors arranged in a spaced-apart relationship at least along an axis of said carrier, such that progression of the probe through a tissue mass provides for locating and determining a dimension of an abnormal tissue specimen inside said tissue mass based on characterization signals from the sensors in the array, thereby enabling consequent treatment of the abnormal tissue specimen by a treatment tool.

US Patent 8,002,713 teaches a biopsy device for tissue collection, the biopsy device includes a biopsy needle module. The biopsy needle module includes a biopsy needle and a cutting sleeve, the biopsy needle having a sharpened distal end and a distal opening for collection of tissue, the cutting sleeve having a cutting blade on its distal end and being coaxially positioned with respect to the biopsy needle.

US Patent 6,083,176 discloses a handle assembly having an opening that allows for insertion of a needle set. The needle set is an integral unit and consists of an outer hollow cannula and an inner pointed tip stylet. The stylet and the cannula are capable of being urged forward separately into the biopsy area in a defined motion in relation to each other. The handle assembly includes a housing, a cannula extension and a stylet extension. In operation, the stylet and the cannula are inserted into the housing. The extensions are slidable and moved rearward separately until the stylet and the cannula are in a spring loaded position wherein first locking members have engaged second locking members on both the stylet and the cannula. The stylet and the cannula are inserted into a patient near the biopsy area. The stylet is then urged into the biopsy area. The stylet extension is pushed forward by a user's thumb and the stylet is fired so that the tissue is pierced. The cannula extension is triggered by the firing of the stylet and automatically urged forward so that the tissue is severed and captured in the notch of the stylet. After disengaging the biopsy area, the stylet is pressed forward using the extension of the stylet so that the tissue sample is exposed and may be removed. The stylet and cannula are then pulled back into the starting position so that multiple samples may be taken.

### GENERAL DESCRIPTION

The present invention provides a novel and effective technique in biopsy procedures, by accurately sampling tissue in vivo including correctly identifying tissue of interest, e.g. tissue suspected as being abnormal or diseased and effectively collecting the tissue of interest, while at the same time minimizing unwanted extraction of healthy tissue in proximity to a tissue of interest. Additionally, the present invention enables precisely locating tissue of interest by providing a novel tissue characterization technique while enabling sampling only the tissue of interest and keeping other tissue in place.

The present invention provides fast, easy to implement and accurate tissue sampling, and obtaining a "high-quality" and undamaged tissue sample, in order to optimize subsequent laboratory examination procedures.

The present invention provides a novel biopsy device which precisely collects from a body an exact tissue portion which is effectively diagnosed and located. The biopsy device of the invention enables immediate sampling of the same tissue which has been characterized by the device. This is achieved by performing two subsequent actions, in a very short time, on exactly the same tissue portion while keeping the biopsy device (its tissue collecting part) stationary in the same place, with no need to move it either axially or rotationally during the collection action that follows the examination action. The biopsy device of the invention has a tissue characterization mode, in which tissue in contact with the biopsy device, typically at a distal portion thereof, is continuously characterized as the biopsy device is moved within the tissue. If the properties of the characterized tissue portion meet the criterion looked for, the biopsy device actuates a collection mode by cutting only the tissue portion which has just been examined and characterized as a tissue of interest. This results in high quality tissue samples while reserving unneeded tissue. At the same time, the process is very fast and convenient involving a single act from a user (human or machine-controlled) to collect the tissue portion.

Thus, according to a first broad aspect of the invention, there is provided a biopsy device comprising an inner needle, and outer sheath and a tissue sensing unit configured to sense tissue properties, mounted on the outer sheath. The inner needle comprises a tip configured to pierce tissue, and a cavity configured for receiving a tissue portion from a region of interest. The outer sheath is configured to move with respect to the inner needle between a forward position of the sheath in which it totally covers the cavity and a backward position of the sheath in which it totally reveals the cavity, or in other words, the cavity is exposed or is totally uncovered by the sheath. The outer sheath comprises a cutting edge at its front end configured to cut said tissue portion into said cavity while the sheath moves in a forward direction, from its retracted (backward) position to its forward position covering the cavity. When the outer sheath is in the forward position the tissue sensing unit is aligned, both axially and azimuthally, with the cavity in the inner needle, to thereby sense signals indicative of tissue properties (characterize tissue) from the tissue portion that is adjacent to (above) the location of the cavity. That is, the tissue portion being a candidate to be cut. That is, the tissue portion that will be cut into the cavity when the sheath is moved to its backward position, to expose the cavity, and then back to its forward position, to cut the tissue and keep it secured in the covered cavity.

Generally, the biopsy device of the invention is operated as follows. The biopsy device is inserted into a region of interest while in its closed configuration, i.e. the sheath is fixedly secured in the forward position. The biopsy device is moved inside the region of interest while the tissue sensing unit continuously scans the tissue above/adjacent to the location of the sampling cavity, and provides indications on the instantaneous properties (e.g., electrical properties) of this tissue. When the user detects a suspected tissue, he activates, while the biopsy device is maintained in place, a tissue sampling process in which the sheath is moved backward to open the cavity, allowing the suspected tissue to enter the cavity, and immediately after the sheath is moved (back) forward to cut the suspected tissue, thus leaving the cut tissue inside the cavity. The backward and forward movement (which may be referred to herein as reciprocal movement) of the outer sheath is performed/actuated as a single continuous process.

In some embodiments, the tissue sensing unit comprises an array of spaced-apart tissue characterization sensors arranged above and along the cavity when the sheath is in the forward position.

In some embodiments, the tissue sensing unit can be integral with the sheath.

In some embodiments, the tissue sensing unit comprises near-field electromagnetic sensors.

In some embodiments, the tissue sensing unit comprises capacitance sensors.

According to a second broad aspect of the present invention, the biopsy device comprises an inner needle, an outer sheath and a vacuum/suction mechanism by which a suction force is continuously applied to the sampling cavity in the inner needle. When the biopsy device is in the closed state, negative pressure, relative to the pressure outside, builds up within the sampling cavity. When the tissue sampling process is initiated, and the outer sheath is retracted/moved backwards, the negative pressure aids in effectively pulling the tissue into the sampling cavity. This assists in obtaining high-quality tissue samples, which fill the tissue-collecting cavity. The suction/vacuum is generally applied from the backside of the cavity farthest away from the needle's tip. This assists in maintaining negative pressure within the sampling cavity throughout the whole phase/time during which the outer sheath is retracted. The biopsy device may optionally comprise the tissue properties sensing unit mounted on the outer sheath which operates as described above.

According to the invention, the biopsy device comprises a movement mechanism responsible for the consecutive revealing and covering of the collecting cavity, obtained by backwards and forwards movements of the sheath. The movement mechanism is connected to the sheath at a backside thereof and configured for controllably moving the sheath, in a continuous manner and in response to a single activation, firstly in a backward direction from said forward position to said backward position, and secondly in said forward direction from said backward position to said forward position.

The movement mechanism can comprise a locking mechanism comprising a slider and a latch configured respectively to enable retraction and fixation of said sheath into said backward position, to thereby enable extraction of said tissue portion from said cavity.

In some embodiments, the movement mechanism comprises a spring which is manually energized prior to activating movement of said sheath.

In some embodiments, the movement mechanism comprises at least first and second springs, said first spring is energized prior to activating movement of said sheath to thereby move said sheath in the backward direction to said backward position, said second spring is energized by relaxation movement of said first spring to thereby move said sheath in the forward direction, from said backward position to said forward position.

In some embodiments, the movement mechanism comprises at least first and second springs both of which are energized prior to activating movement of said sheath, relaxation movement of said first spring causes movement of said sheath in the backward direction to said backward position, followed by disengagement of said second spring which relaxation movement causes movement of said sheath in the forward direction, from said backward position to said forward position.

In some embodiments, the movement mechanism comprises a Scotch yoke mechanism comprising a torsion spring configured to be energized and to attach during relaxation to a rotating disk of the scotch yoke mechanism, relaxation movement of said energized torsion spring causes movement of said sheath in the backward and forward directions.

In some embodiments, the movement mechanism comprises a torsion spring configured to be energized and a piston engaged at one side with the torsion spring and at a second side with said outer sheath, relaxation movement of said energized torsion spring causes movement of said sheath in the backward and forward directions via said piston.

In some embodiments, the movement mechanism is configured to move said outer sheath and inner needle together forwardly while keeping the cavity covered by the outer sheath. The movement mechanism can comprise a spring and a pin, relaxation of the spring causes said outer sheath and inner needle to move forwardly, said pin is attached at one point to said inner needle and engages at another point, during movement, with the outer sheath, to thereby prevent relative movement between the outer sheath and inner needle while moving together forwardly.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Figs. 1A-1D** schematically illustrate schematic side and top views of an embodiment of a biopsy device configured according to the invention comprising a tissue sensing unit;
**Figs. 2A-2B** show an isometric view of an example of a biopsy device of the invention carrying a non-limiting example of a tissue sensing unit which is specifically shown in an enlarged view in Fig. 2B; **Fig. 2C** show a schematic side view of a non-limiting example of a biopsy device of the invention comprising an outer sheath having a slanted cutting edge;
**Figs. 3A-3D** schematically illustrate another embodiment of a biopsy device configured according to the invention comprising a suction system;
**Figs. 4A-4L** exemplify one embodiment of a movement mechanism for use with a biopsy device of the invention;
**Figs. 5A-5K** exemplify a second embodiment of a movement mechanism for use with a biopsy device of the invention;
**Figs. 6A-6C** exemplify in an enlarged view of the location and mode of action of levers for actuating the various movement steps of the movement mechanism, and a locking mechanism for use with a biopsy device of the invention;
**Figs. 7A-7H** exemplify a third embodiment of a movement mechanism for use with a biopsy device of the invention;
**Fig. 8** exemplifies a fourth embodiment of a movement mechanism for use with a biopsy device of the invention;
**Fig. 9** exemplifies an embodiment of a movement mechanism where the needle and sheath are capable of advancing together for use with a biopsy device of the invention and
**Figs. 10A1-10E2** exemplify another embodiment of a movement mechanism where the needle and sheath are capable of advancing together for use with a biopsy device of the invention.

### DETAILED DESCRIPTION OF EMBODIMENTS

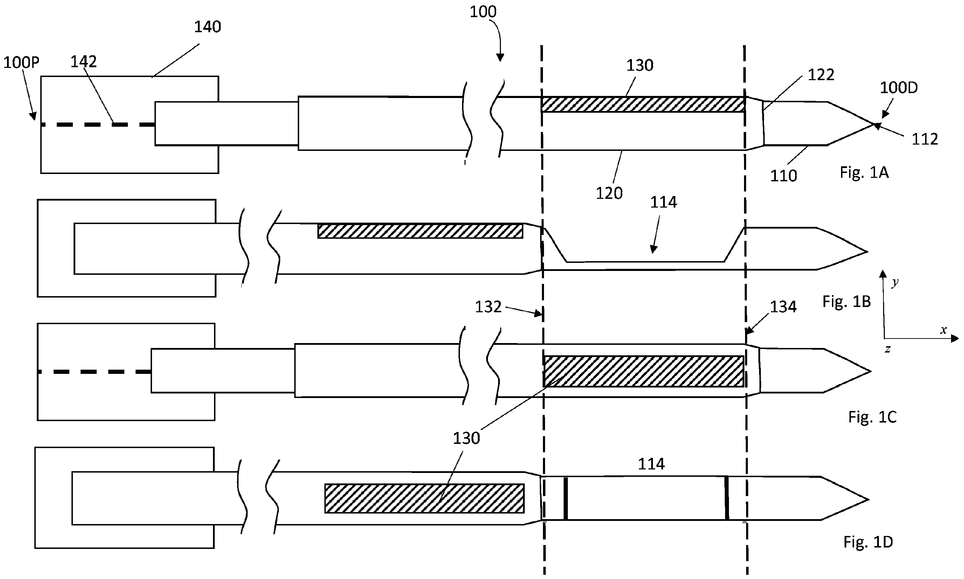

Reference is made to **Figs. 1A-1D** showing a first non-limiting example of a biopsy device **100** configured in accordance with the present invention. **Figs. 1A-1B** are side views of the biopsy device in its closed and open positions respectively. **Figs. 1C-1D** are top views of the biopsy device in its closed and open positions respectively. As seen in the figures, the biopsy device **100** includes an inner needle **110** comprising a tip **112,** at the distal end **100D** of the device, which is configured to pierce tissue while moving inside a region of interest in a biological body. As shown in the open configurations in **Figs. 1B and 1D**, the needle **110** also includes a sampling cavity **114** which is configured to receive a tissue portion from the region of interest after being cut by the biopsy device **100** as will be explained below.

The biopsy device includes an outer, coaxial, sheath **120** which is configured to move with respect to the inner needle **110** between a forward position, or in other words a closed position/configuration, and a backward position, or in other words an open position/configuration. As shown in **Figs. 1A and 1C****,** the outer sheath **120** covers the cavity **114** when it is in its forward position. Additionally and preferably, in this state the outer sheath **120** seals the cavity **114** such that passage of fluid or any other substance from the region of interest and the cavity **114** is restricted. To this end, the outer sheath **120** or the inner needle **110** may include sealing means, such as a ring, which insures the cavity is sealed, as will be exemplified below. In the backward/open position, as shown in **Figs. 1B and 1D****,** the outer sheath **120** uncovers the cavity completely by moving backwards with respect to the inner needle **110.** During the backward movement of the sheath, the tissue portion adjacent the revealed cavity enters the cavity **114.**The outer sheath **120** includes a cutting edge **122,** at its front side, which is configured to cut the tissue portion that is in the cavity **114** while the outer sheath **120** moves in a forward direction from the open state to the closed state.

The biopsy device **100** also includes a tissue sensing unit **130** which senses tissue properties and characterizes the tissue, e.g. identifies abnormal or diseased tissue. The tissue sensing unit **130** is mounted on the outer sheath **120,** at a distal portion thereof, such that the tissue sensing unit **130** is aligned with the cavity **114** when the sheath is in the forward (closed) position, i.e. the tissue sensing unit extends above the cavity **114** between the edges of the cavity **114,** as partially illustrated by the dashed lines **132** and **134.** While the dashed lines **132** and **134** illustrate the alignment in the longitudinal axis ***x*** along the length of the biopsy device **100,** it should be understood that the alignment between the tissue sensing unit **130** and the cavity **114** is maintained also along the width of the device, i.e. in the azimuthal direction as well, as shown in **Figs. 1C**. In other words, the area sensed by the tissue sensing unit **130** matches the area of the tissue portion that will be received into the cavity **114** when the sheath is retracted backwards, and will be cut when the sheath is moved back forwards to its forward (closed) position. The design of the tissue sensing unit **130,** the movement of the outer sheath **120** relative to the needle **110,** and the cavity **114** is made to insure that only the tissue portion sensed is cut and received in the cavity **114.**

In this example, the biopsy device **100** further includes a handle **140** at its proximal end **100P** configured, inter alia, to enable effective and comfortable gripping of the device **100** by a user.

It should be noted, that throughout the current application, the words "forward" and "distal" and "right (of the page)" at one hand, and the words "backward" and "proximal" and "left (of the page)" at the other hand, and their corresponding derivatives, may be used interchangeably.

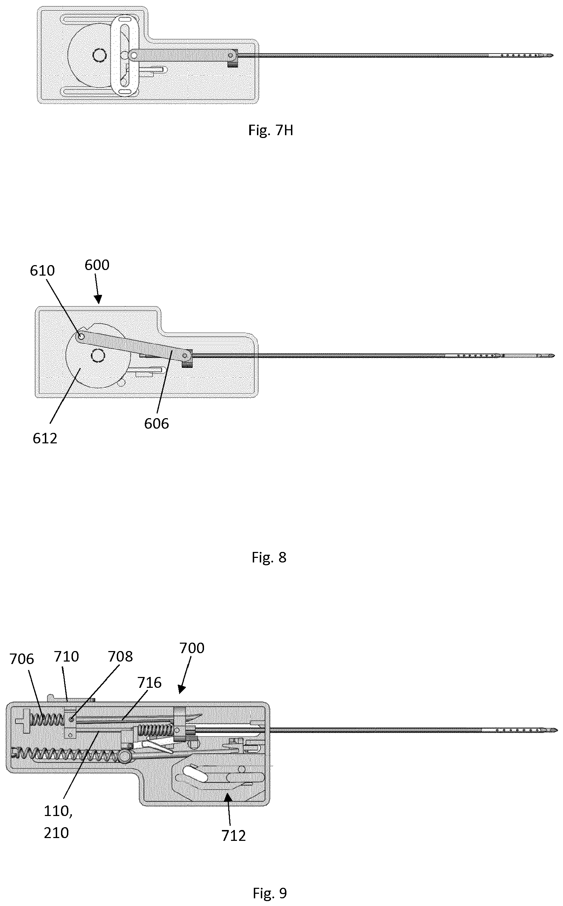

The biopsy device **100** can include a movement mechanism, as described below, which enables the above-mentioned movement of the outer sheath **120** backwards and forwards between the forward and backward positions. The movement mechanism can be accommodated inside the handle **140.** It is appreciated that the handle **140** and the inner needle **110** do not have relative movement there between, apart from some examples showed below, and accordingly are firmly attached or removably fixed to each other as illustrated by a dashed link **142.** It should be understood that the fixation of the inner needle **110** to the handle **140** may be at any suitable point in the handle as implied by the design and function of the handle's internals, as will be described further below. Alternatively, the inner needle may be configured to move relative to the handle as will be further detailed with reference to Fig. 9.

Generally, the biopsy device **100** has a hollow cylindrical-like outer sheath **120** and a matching smaller and enclosed full-bodied or semi-full cylindrical-like inner needle **110** with the exception of the cavity **114** formed in the needle **110.** As such, the biopsy device **100** is round across the transverse section, i.e. in the *y*-*z* plane, with a circular or semi-circular cross-section, as will be shown in some embodiments below.

The needle's tip **112** is configured for easy, convenient and effective piercing force. In some embodiments, the tip **112** has a conical shape with a round base matching the overall transverse cross-section in the *y*-*z* plane of the needle **110.** In some other embodiments, the tip **112** may be formed as a pyramid-like shape with edges between the pyramid faces that function as cutters which improve the tissue penetration. The angle of the tip's apex is optimized to insure convenient and less painful / less harmful penetration procedure. The tip **112** is also sealed such that no tissue is collected at any point in the biopsy device apart from the upper side of the cavity **114** when it is revealed by the backward movement of the outer sheath **120.**

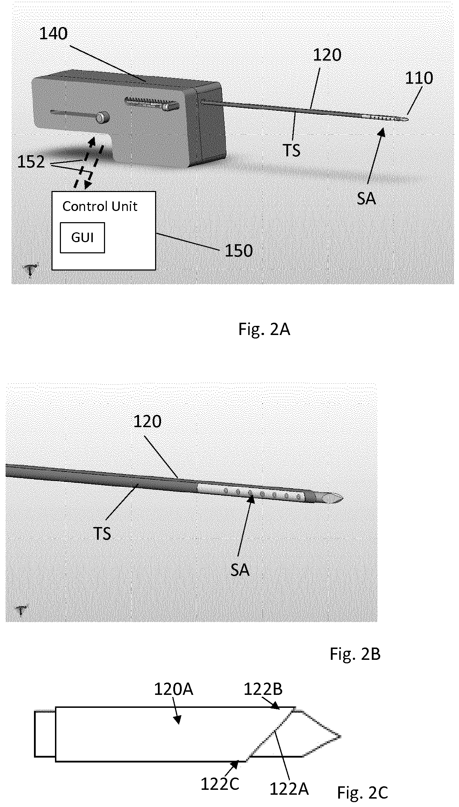

The tissue sensing unit **130** is designed to sense the properties of a tissue portion that comes in contact with the tissue sensing unit **130.** The tissue sensing unit **130** is preferably configured to operate continuously and to provide instant and continuous sensing data from the tissue, i.e. to scan the tissue portion it contacts. During operation, the signals indicative of the tissue properties obtained by the tissue sensing unit **130** are received continuously in a control unit (as shown in Fig. 2A) which is connected in a wired or a wireless connection to the biopsy device **100.** The control unit analyzes the tissue signals data on-line and presents an output data indicative of the tissue type or characteristics to the user.

It should be noted, that when the outer sheath **120** is in its forward (closed) position, the biopsy device is in a "scan mode" in which the biopsy device scans the tissue by the sensing unit **130.** The biopsy device **100** is typically equipped with a locking mechanism that keeps the outer sheath fixed in the closed state as long as the scan mode lasts, thus preventing any accidental and unintended displacement of the tissue sensing unit **130** during the scan mode.

The tissue sensing unit **130** includes sensor arrangement of one or more sensors of any of the known in the art sensor types being configured for sensing biological tissue properties, such as optical or electromagnetic sensors. The sensor(s) and the communication line(s) connecting the sensor(s) to a control unit are preferably flat and thin, so that they could be accommodated on the outer sheath **130** without substantially increasing the diameter of the outer sheath, and without creating resistance to motion as the biopsy device is moved within the tissue. Specifically, the tissue sensing unit **130** may be configured according to embodiments described in WO2011016035, titled "Electromagnetic Sensor For Use In Measurements On A Subject", or US20120316463, titled "Medical Device And Method For Use In Tissue Characterization And Treatment" , both assigned to the assignee of the present application. In one specific non-limiting embodiment, as illustrated in **Figs. 2A-2B****,** the tissue sensing unit includes the sensor arrangement **SA** as an array of flat and thin tissue characterization sensors **SA,** seven such sensors are exemplified. The array of sensors **SA** may be arranged along a predetermined axis, here the axis *x*, in a spaced-apart relationship, such that they provide tissue signals from a tissue portion which may be cut by the sheath's cutting edge **122** and received in the needle's cavity 114 upon identifying tissue properties which are of interest during the biopsy procedure. **Fig. 2B** is a magnified view of the tissue sensing unit including the flat and thin array of sensors **SA** and the communication line (Transmission Structure **TS**). In some embodiments, the tissue sensing unit **130** may include a two-dimensional array of spaced-apart sensors, e.g. a matrix of sensors. The two-dimensional array is aligned with the sampling cavity in the inner needle, and may be spatially organized to cover, or partially cover, a portion of the circular side wall of the cylindrical sheath **120.** In yet another embodiment, the tissue sensing unit **130** may include a capacitance, also termed capacitive, sensor, or a spaced-apart array of capacitance sensors.

In some embodiments, the tissue sensing unit **130** may be embedded in the outer surface of the outer sheath **120,** e.g. in specially designated aperture(s), thus forming an integral part of the outer sheath **120** and the biopsy device **100,** such that it does not protrude outwardly from the outer sheath's surface (side wall) and does not add any bulk volume to the biopsy device. The array of sensors **S** is connected by a flexible and flat signal transmission structure **TS,** as also taught by WO2011016035 to a suitable control unit (not shown) for sending sensing data thereto.

The control unit **150** is connected to the biopsy device (e.g. at the side of the handle 140) via a wired connection/ a cable set **152** to the biopsy device, and to the sensors **SA** through the transmission line **TS** that is connected to the cable set **152.** The control unit continuously sends signals to the sensor arrangement and receives on-line signals from the sensor arrangement, i.e. signals originating from the concrete location of each sensor at each position of the biopsy device inside the tissue mass. The signals sent and received by the control unit **150** include tissue characterization signals, e.g. electromagnetic fields which are transmitted to the sensors **SA** and reflected back from the sensors **SA,** the amplitude and phase of the reflected electromagnetic fields being dependent on the electrical properties of the tissue in close proximity to the sensors SA. The control unit can further analyze the signals and display the signals or a tissue profile of the measured parameter on the included graphical user interface (GUI). The control unit can be preprogrammed to receive signals only when the outer sheath is in the forward direction, i.e. when the cavity is closed, or it can receive all signals and discard the signals arriving during the collection phase, i.e. during the backwards and forwards movement of the outer sheath. The user can make a decision whether to activate a tissue collection mode at a specific location of the biopsy device inside the tissue, based on the signals received in the control unit. Additionally or alternatively, the control unit **150** can generate output data being indicative of a condition of the measured tissue, and by this indicate whether a tissue collection mode should be carried out at a specific position of the biopsy device inside the tissue.

Reference is made to **Fig. 2C** showing a non-limiting example of a specific outer sheath **120A** having a slanted/inclined cutting edge at the distal end thereof. As shown, the cutting edge **122A** is slanted, angled, such that the sheath is longer at the side **122B** of the cavity, and shorter at the opposite side **122C.** This specific structure makes the piercing and cutting of the tissue more efficient, because the resistance of the tissue to the progressing outer sheath is less when compared to a cutting edge being vertical to the progression direction.

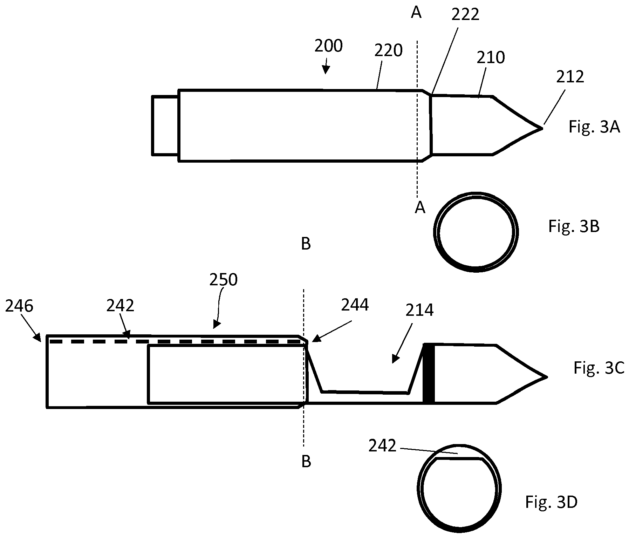

Reference is made to **Figs. 3A-3D****,** illustrating a non-limiting example of another embodiment in accordance with the present invention. A biopsy devise **200** is shown and includes an inner needle **210** and an outer sheath **220.** The device **200** also includes a handle (not shown) as in device **100.** The inner needle **210,** the outer sheath **220** and the handle are similar in their functions to the inner needle **110,** the outer sheath **120** and the handle **140** of the biopsy device **100** described above, however they have some different constructional elements as will be described below. Accordingly, the inner needle **210** includes a tip **212** which is configured to pierce tissue, and a cavity **214** which is configured to receive a tissue portion from the region of interest after being cut by the biopsy device **200.** The outer sheath **220** is configured to move with respect to the inner needle **210** between a forward position, or in other words a closed position/configuration in which the sheath covers the cavity **214** as shown in **Fig. 3A****,** and a backward position, or in other words an open position/configuration in which the sheath uncovers and totally reveals the cavity **214** as shown in **Fig. 3C****.** In the closed position, the outer sheath **220** seals the cavity **214** entirely, in order to prevent or minimize the passage of fluid or any other substance from the region of interest to the cavity **214.** This is illustrated in **Fig. 3B** which is a view of the transverse cross section along the dashed line **A-A**. As shown in **Fig. 3B**, both the cross sections of the inner needle **210** and the outer sheath **220** are circular in order to prevent or minimize the passage of matter or fluid (gas or liquid) between them because of them being close in their diameters. To this end, the outer sheath **220** or the inner needle **210** may include sealing means which assists in sealing the cavity when the sheath **220** is in the forward position. For example, a sealing member **218** is shown in **Fig. 3C****,** wrapping the outside of the inner needle **210**. Alternatively, a sealing means can be attached firmly to the inner lumen of the outer sheath at the distal side thereof. The outer sheath **220** includes a cutting edge **222,** at its front (distal) end, which is configured to cut the tissue portion into the cavity **214** while the outer sheath **220** moves in a forward direction from the open state to the closed state.

The biopsy device **200** includes a suction system **250** which is configured to apply suction force in the cavity **214** to thereby pull the tissue portion in the vicinity of the cavity, usually above the cavity, into the cavity while the sheath **220** moves in the backward direction, i.e. during the process of uncovering the cavity **214.** The pulling of the tissue portion into the cavity **214** improves the quality (size and integrity of the cut tissue sample), and tissue cutting with the cutting edge **222** of the outer sheath **220** while the latter moves forward towards its closed position.

Typically, the suction system **250** includes a hollow channel **242** which is formed / built in a space between the inner needle and outer sheath, as shown by the dashed line in **Fig. 3C** exemplifying the itinerary/route of the suction/vacuum applied, and by the channel/space **242** shown in **Fig. 3D** which is a cross section along the dashed line **B-B**. From the location of section line **B-B** towards the proximal (handle side) end of the inner needle, the cross section of the inner needle **210** is not circular, instead it is truncated at one side at least in order to form the vacuum channel **242** between the inner needle and the outer sheath. The vacuum channel, which might be also applied using a hollow conduit/tube, runs through the biopsy device **200** such that it is connected at its one side **244** to the cavity **214** and at its second side **246** to a suction pump (not shown) which is usually positioned outside the body/examined tissue, e.g. inside the handle of the device, or within the control unit (such as the control unit shown in Fig. 2A). Preferably, as shown in the figures, the suction system **250** is connected to the back side of the cavity **214** at the far point from where the cavity starts to get uncovered by the backward movement of the outer sheath **220.** By this, the pulling force acting on the tissue resulting from the vacuum/suction is maintained inside the cavity when the outer sheath moves backwards, thereby enhancing the tissue entry into the cavity.

The suction force applied by the suction system **250** is typically achieved by generating negative pressure inside the cavity **214** relative to pressure in the tissue surrounding the biopsy device **200**.

It should be understood, that in some non-limiting embodiments, not specifically shown in **Figs**. **3A-3D****,** the biopsy device **200** additionally includes a tissue sensing unit being configured as described above with reference to **Figs. 1A-1D** **and** **2.**

In order to collect tissue, the biopsy device of the present invention includes an innovative movement mechanism responsible for the continuous, backwards and forwards (reciprocal), movement of the outer sheath relative to the inner needle, such that the cavity is gradually revealed to receive a tissue portion which is subsequently cut by the movement of the sheath in the forward direction.

The movement mechanism is connected to the outer sheath, generally at its backside (at a proximal end of the sheath), and is configured to move the outer sheath, in a continuous manner and in response to a single activation by the user, firstly in a backward direction from the forward position to the backward position, and secondly in the forward direction from the backward position to the forward position.

Additionally, the movement mechanism includes an option to hold the cavity revealed in an open position so to enable the withdrawal of the collected tissue, when the device is outside the body. In other words, the movement mechanism enables retracting the outer sheath and keeping it fixed in the backward position so that the cut tissue portion can be safely extracted from the cavity.

Additionally, the movement mechanism can include an option to move both the inner needle and the outer sheath forwardly together, i.e. to fire both of them in the distal direction, while the cavity in the inner needle is kept covered by the outer sheath. This common forward movement can be advantageous when there is a need to effectively/forcefully pierce a tissue mass before starting the characterization and sampling procedures.

It should be noted that the movement mechanism is typically mechanical though it can be electrically implemented using a dedicated electrical motor responsible for moving the outer sheath in both directions, backwards and forwards, in a controlled manner. The latter configuration is not specifically described in details in the application, it typically involves the motor and a controller which controls the speed of the motor which moves the outer sheath. One advantage for using an electrically controlled motor is the excessive control over the sheath's movement. For example, electrical control enables moving the outer sheath a partial way between its backward and the forward positions, or moving the sheath in a controllable, e.g. variable, speed, in the forward and backward directions.

In the following, several embodiments of the movement mechanism are described. The described movement mechanisms can be used with any device configured according to the present invention, e.g. the above-illustrated devices **100** and **200** or a combination thereof.

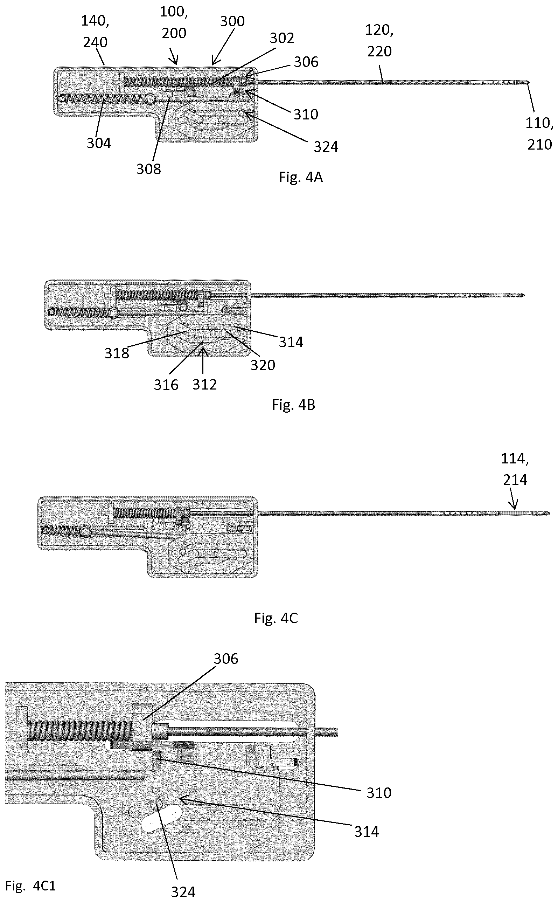

Reference is made to **Figs. 4A-4L** illustrating a first example of a movement mechanism **300** in accordance with the present invention. The movement mechanism is used in a biopsy device (**100,200**) which includes the inner needle (**110, 210**), the outer sheath (**120,220**) and the handle (**140,240**). The movement mechanism is located inside the handle and is configured to move the outer sheath (**120,220**) backwards, totally revealing a cavity (**114,214**) in the inner needle to receive a tissue portion therein, and then forwards to its closed position, causing cutting of the tissue portion and enclosing it inside the device. The figures 4A-4H illustrate the various movement stages of the outer sheath.

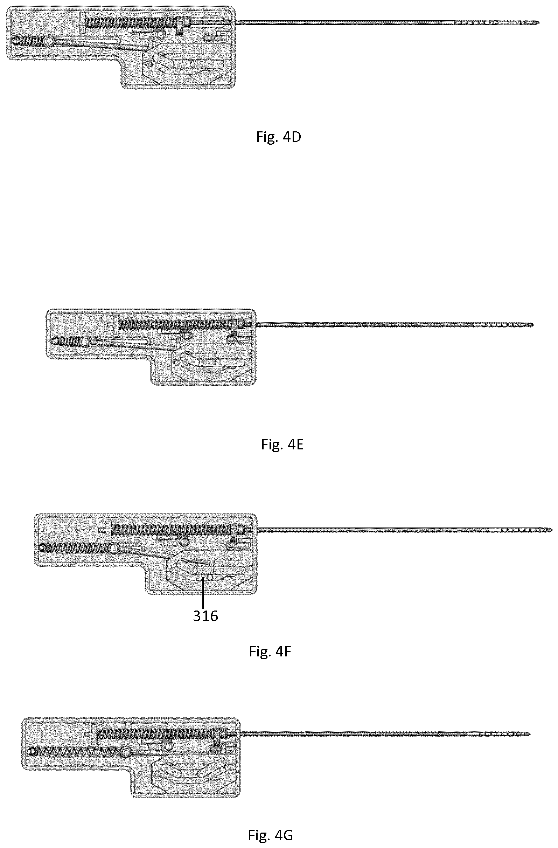

The movement mechanism **300** includes two springs **302** and **304** configured to move the outer sheath in the backward and forward directions. In order to prepare for the movement cycle and initiate the continuous movement, backwards and forwards, the movement mechanism includes handles/sliders and buttons in the handle (**140,240**) which enable a user to energize one or both springs and to release at least one energized spring to cause the desired movement. In this example, the spring **302** is connected to the sheath, by a coupler **306** which is fixedly connected to the backside of the sheath, and moves with the sheath backwards and forwards. The spring **304** is firmly connected to a pin **308** that engages with the backside of sheath (**120,220**). This is achieved by a protrusion **310** at the distal end of the pin **308** that pushes against the coupler **306.** **Fig. 4A** shows the conditions before starting the movement, when the biopsy device is in scan mode. The spring **302** is relaxed, the spring **304** is energized by extension, the pin **308** is locked in its forward position, and the sheath is in its forward position being locked in its forward position. The user releases a latch (not shown) that holds the pin **308** and consequently the spring **304** in the described conditions (the latch also locks the sheath in the forward position), and the movement backwards starts. As shown in **Fig. 4B****,** the spring **304** starts its relaxation to the left pulling with it the pin **308,** and the protrusion **310** which is in contact with the coupler **306** thereby pushing the coupler **306** to the left. The sheath which is attached to the coupler also moves backwards thereby exposing the sampling cavity. Tissue adjacent the sampling cavity enters and fills the cavity. During this backward motion, the spring **302,** which is fixedly attached to the coupler **306,** is compressed and energized. The pin **308** is fixedly connected at its distal end to another pin **324** which moves in a defined and constricted closed route **312** such that it travels in different paths/slots during the backward and forward movements. Technically, the route **312** includes two paths **314** and **316,** and levers **318** and **320** at the end of each path respectively that allow movement of the pin **324** in one way only. During its backward movement, the pin **324** travels along the upper straight path **314.** In **Fig. 4C****,** the backward movement of the sheath is completed and the pin **324** has traveled to the far most left point of the straight upper path **314.** At its far most left end, the path **314** twists downwards and the pin **324** travels down causing the protrusion **310** to detach from coupler **306** and thus from the spring **302** which is now energized in compression. This is further illustrated in **Fig. 4C1** showing a close up view of the spatial relation between the pin **324,** protrusion **310** and coupler **306** at the proximal part of the path **314.** The cavity (**114,214**) is fully revealed. In **Fig. 4D****,** the spring **302** which is now released and energized starts to extend to the right towards its relaxed position. During this motion, the spring **302** pushes the sheath forwards back to the forward position. During this forward motion of the sheath, the tissue which has entered the cavity is cut and maintained within the cavity. The end of the forward movement is shown in **Fig. 4E****,** with the sheath back in its forward position, fully covering the sampling cavity. As shown in **Fig. 4D and Fig. 4E****,** during the forward movement of the spring **302,** the spring **304** is relaxed and stays stationary in its back position.

An additional handle/slider (not shown) is then used to manually move the outer sheath to its backward position, against the spring **302**'s force, and to lock it (by use of a dedicated latch, not shown) in this backward position, thereby exposing the cavity so that the tissue portion within the cavity may be extracted. In order to start another movement cycle, while the sheath is locked in its backward position, the spring **304** is energized manually. In this connection, it should be noted that the back-locking feature of the outer sheath is possible with every illustrated example of the movement mechanism of the invention, though it is not specifically shown with every example.

**Figs. 4F-4H** illustrate the manual energization of the spring **304.** By utilizing a suitable handle/slider (not shown), the user pushes the handle forwards causing the pin **324,** attached to pin **308,** to travel along the lower path **316** back to its position **322.** The pin **324** cannot travel in the upper path **314** because of the lever **318** which blocks movement in the forward direction. Following this, the sheath is released from its backward position, and the device is back in the state as described in **Fig. 4A****,** ready for the next backwards and forwards movement cycle.

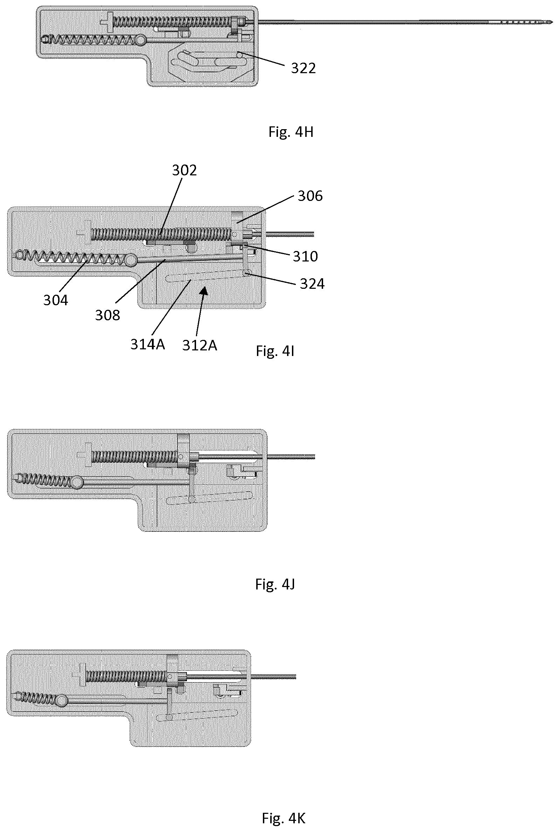

As appreciated, **Fig. 4H** illustrates the same condition as Fig. 4A to enable another sheath movement cycle backwards and forwards.

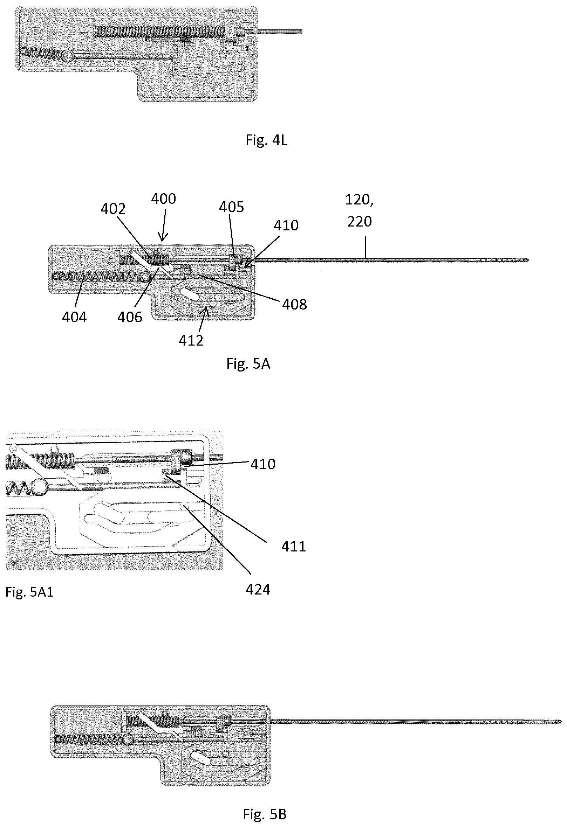

Turning to **Figs. 4I-4L****,** there is shown an alternative configuration to the route travelled by the pin **324** during the movement cycle composed of the reciprocal (backwards and forwards) movement of the outer sheath. As appreciated, the pin **324** travels along the same route in the backward and forward direction, and not in separate paths as with the route 312 described above. The orientation of the pin **308** is slanted in relation to that of the outer sheath during the movement cycle. As shown in **Fig. 4I**, a slanted unitary route **312A** is formed by an inclined straight path **314A,** such that when the pin **324** moves to the left, pulling with it the outer sheath backwardly, it moves downwardly at the same time, as in **Fig. 4J**, so to gradually go down far from the coupler **306,** until it reaches the most left lower point in the route **314A** at which it has detached from the coupler **306,** as shown in **Fig. 4K****.** The route's inclination angle, the route's length, and/or the length of the relaxed spring **304** are set so that the protrusion **310** is not in contact anymore with the coupler **306** when the outer sheath reaches its backward position. When this happens, the energized compressed spring **302** is free to relax thus pushing the outer sheath to the right forwardly back to its forward position, as shown in **Fig. 4L****.**

An additional handle/slider (not shown) in then used to manually move the outer sheath to its backward position, against the spring **302**'s force, and to lock it (by use of a latch, not shown) in this backward position, thereby exposing the cavity so that the tissue portion within the cavity may be extracted. In order to start another movement cycle, while the sheath is locked in its backward position, the spring **304** is energized manually by pushing the pin **324** to the right up along the route **314A.** Following this, the sheath is released from its backward position, and the device is back in the state as described in **Fig. 4I**, ready for the next backwards and forwards movement cycle.

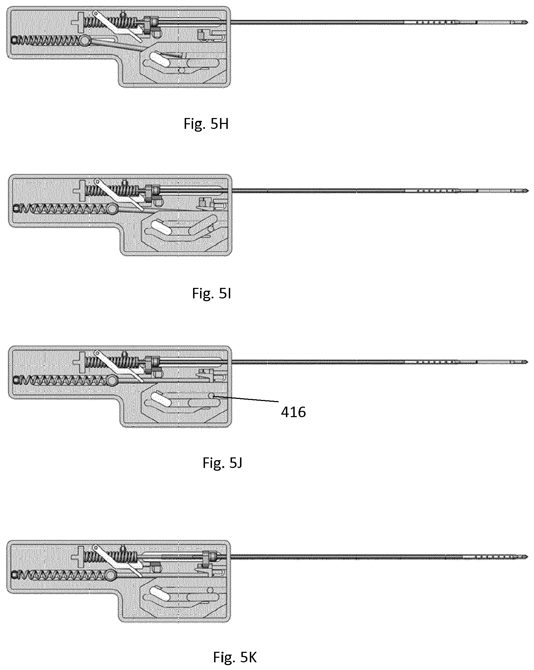

Reference is made to **Figs. 5A-5K****,** illustrating another non-limiting example of a movement mechanism **400** according to the present invention.

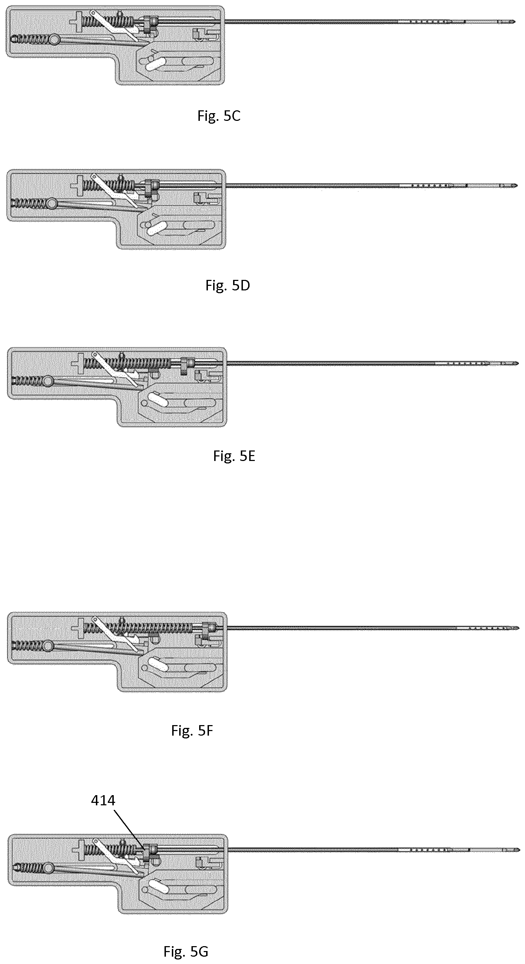

In this movement mechanism, a spring **402** is not fixedly connected to the sheath. The sheath is fixedly attached at its proximal (back) side to a coupler **405,** in the same configuration as coupler **306.** **Fig. 5A** shows the state of the movement mechanism prior to starting the movement cycle. The two springs, **402** and **404** are initially energized prior to starting the movement cycle, and not only one spring as in the previous example. The spring **402** is energized in compression and held back by a hinge **406** which is not fixedly connected to the spring. The spring **404** is energized in extension and fixedly connected to a pin **408** which engages with the back end of the outer sheath (**120,220**) by the protrusion **410** at the distal side of the pin **408.** Further detail is provided in **fig 5A1**. The protrusion **410** is in contact with the back side of the sheath via the coupler **405,** such that it pulls the coupler **405** backwards during the backward movement. An additional protrusion **411** is provided in order to release the hinge **406** when the pin **408** is moved backwards, as will be further detailed below. During the movement in both directions, the pin **408** moves inside a closed route **412** configured exactly as route 312 described above. This is achieved, in the same manner as in the example above, by a pin **424** which is fixedly connected to the pin **408.** In **Fig. 5B****,** the pin **408** is released by pushing on a dedicated button in the handle (not specifically shown) of the device. The spring **404** starts to relax backwards to the left pulling with it the outer sheath. In **Fig. 5C****,** the pin **408** starts to move downwards inside the route **412** in the same manner described in the example above. Alternatively, the pin 408 may move in an inclined route as route 312A, as described above in Figs. 4I-4L. The completion of the downward movement of the pin **408** is depicted in **Fig. 5D****.** In this condition, the sheath is in its backward position, and the sampling cavity is fully exposed (uncovered). The completion of the downward movement of the pin **408** causes two movement actions; it detaches the pin **408** from the outer sheath (as protrusion 410 is not in contact anymore with coupler 405), and pushes the hinge **406** (via protrusion 411) which as a result releases the compressed spring **402.** When the spring **402** is released, it relaxes to the right thus pushing the outer sheath forwards, as shown in **Figs. 5E** and **5F****.** It is noted again that the spring **402** is not firmly attached to the outer sheath. As shown in **Fig. 5F****,** the sheath is back at its forward position, and the sampling cavity is full covered. **Figs. 5G-5K** illustrate the energizing process of both springs **402** and **404,** so that another movement cycle can be performed. In **Fig. 5G****,** the spring **402** is energized by compression by sliding backwards a dedicated handle/slider **414** which is fixedly attached to the outer sheath. The sliding handle **414** is held, e.g. by means of a latch (not shown), in the backward position compressing the spring **402** and exposing the sampling cavity so that the tissue sample may be extracted. Following this, as shown in **Fig. 5H****,** the user moves the pin **408** forwards in the lower path of the route **412** causing that the pin **408** releases the hinge **406** which was held down by the pin **408,** and the hinge **406** moves back upwards to hold the compressed spring **402** in place. **Figs. 5I-5K** illustrate the movement of the pin **408** forwards inside the route **412,** accompanied with energization of the spring **404** connected thereto, until the pin **408** reaches its most front position at **416** and engages in place contacting the back end of the outer sheath to thereby enable another movement cycle to be carried out..

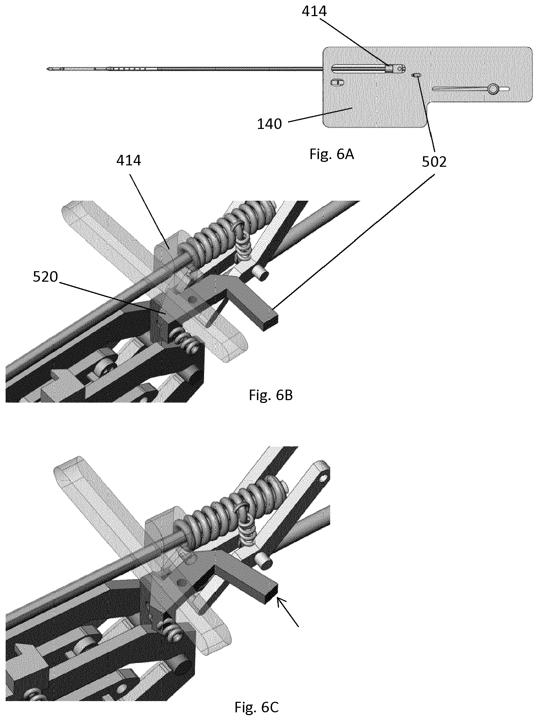

According to the invention, it is important for the movement mechanism to enable retraction and fixation of the outer sheath in the backward position to enable access to the cavity in the inner needle and extraction of the tissue portion from there. This can be implemented by any movement mechanism described herein, by providing a suitable sliding handle, connected to the outer sheath, which can be slid backwards and locked fixedly in a backward position revealing the cavity. As can be appreciated, this feature is inherently implemented in the movement mechanism **400.** Specifically, it is described with reference to **Figs. 5G-5J****.** As mentioned, when the handle/slider **414** is moved backwards in **Fig. 5G****,** it energizes the spring **402** and at the same time it moves the outer sheath connected to it backwards. **Figs. 5H-5J** illustrate the fixedly retracted position of the outer sheath, by providing a locking mechanism (an example is shown in Figs. 6A-6C below), that holds the handle **414** in a back position. Then, after the hinge **406** engages with the compressed spring **402,** as shown in **Fig. 5H****,** the handle **414** is unlocked and disengaged from the back position, thus freeing it and enabling its movement forwards by the user, together with the outer sheath firmly connected to it, as exemplified in **Fig. 5K****.**

Reference is made to **Figs. 6A-6C** showing a non-limiting example of a locking mechanism configured to lock the outer sheath in the backward position. The example utilizes the movement mechanism **400,** however it can be implemented with any other movement mechanism of the present invention. **Fig. 6A** shows a biopsy device according to an embodiment of the invention with a button **502** located in the device's handle **140.** In **Fig. 6B****,** in which the internal side of the handle is shown, the button **502** includes a latch **520** that locks the handle/slider **414,** with the sheath connected to it in a backward position. As has been described, the handle **414** is connected permanently to the outer sheath. **Fig. 6C** illustrates that when the button **502** is pushed (exemplified by the arrow), the latch 520 disengages from the handle **414** which is released and free to be moved along with the outer sheath in the forward direction.

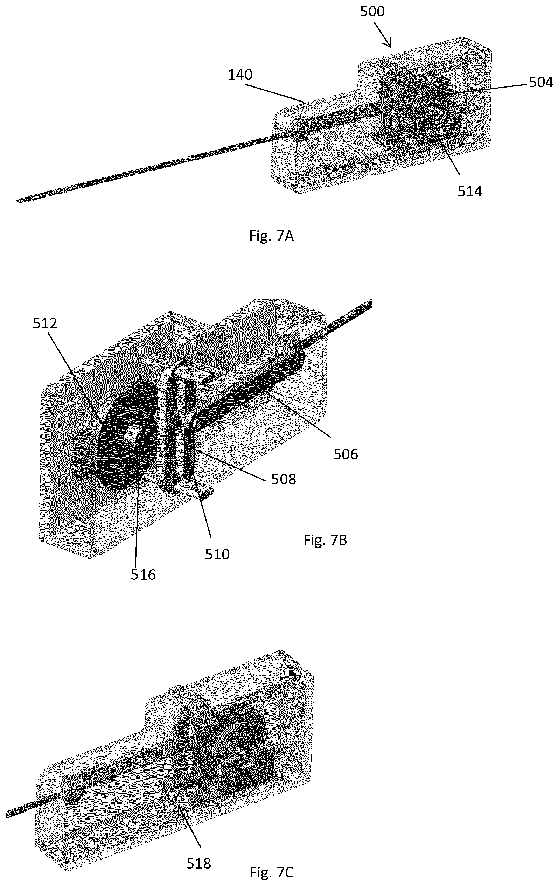

Reference is made to **Figs. 7A-7H** illustrating another movement mechanism **500** according to the invention. This embodiment utilizes a scotch yoke mechanism (slotted link mechanism) that converts a rotational movement to a linear movement (of the outer sheath) and which is well known in the art. As shown in the figures, the movement mechanism **500** includes a torsion spring **504** which engages with and activates the scotch yoke mechanism, the scotch yoke assembly in this example includes a piston or other reciprocating part **506** directly coupled at one side to the outer sheath of the biopsy device and at the other side to a sliding yoke **508** having a slot that engages a pin **510** on a rotating disk **512.** The movement mechanism is activated by energizing the torsion spring **504** by rotating it alone using the handle **514** while the handle **514** is pulled away of the device, and then engaging the energized spring **504** with the rotating disk **512** by pushing the handle **514** inwards such that the teethed disk **516** attached to the spring **504** engages with rotating disk **512.** Releasing the rotating disk **512,** by pushing the button **518,** causes rotation of the rotating disk **512** because of the relaxation movement of the energized spring **504** which is engaged with the rotating disk **512.**

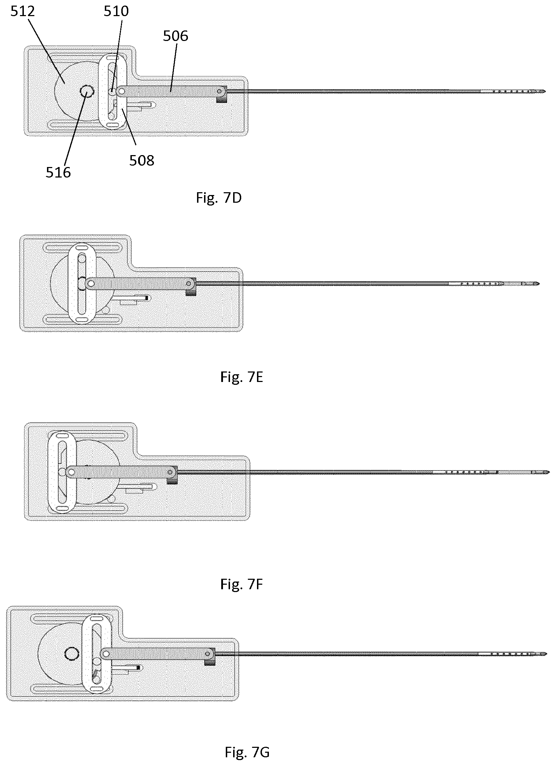

The device includes a stopper (not specifically shown) that insures that the rotating disk **512** rotates for less than a single full circle each time, such that the first half circle moves the outer sheath backwards and the second half circle moves the outer sheath forwards. This is illustrated in **Figs. 7D-7H****.** **Fig. 7D** exemplifies the movement cycle start in which the outer sheath is in the forward position. **Fig. 7E** exemplifies midway in the backward movement towards the backward position of the sheath, such that the pin **510** has moved quarter of the full circle distance. **Fig. 7F** exemplifies the backward position of the sheath, the pin has passed half circle, i.e. half the way. **Fig. 7G** exemplifies the pin **510** just before completing the full circle and **Fig. 7H** exemplifies the return to the start position of Fig. 7D.

As with all other movement mechanisms, the movement mechanism **500** may also include a locking mechanism for locking the outer sheath in the backward position to provide unrestricted access to the cavity in the inner needle.

In yet another embodiment, presented in **Fig. 8****,** the biopsy device may include a movement mechanism **600** based on a torsion spring configured for engaging with a rotating disk **612,** exactly as in movement mechanism **500** described above. The rotating disk **612** has a pin **610** on it to which a straight piston **606** is attached. The rotation of the rotating disk **612** due to relaxation of the torsion spring engaged therewith, causes reciprocal movement of the piston **606** which at its other side is attached to the outer sheath. It is appreciated that this spring-piston mechanism is similar to the scotch yoke mechanism and it is also known in the art.

The movement mechanism can also be configured for additionally moving the inner needle (110, 210), and not only the outer sheath (120,220), relative to the handle (140,240).. For this option, the inner needle and the handle are not fixedly attached as in the configurations described above. In one example, the inner needle and the outer sheath are configured for common forward movement while the outer sheath is in the forward position covering the cavity. This is illustrated in **Fig. 9****,** in which the inner needle can be controllably urged or moved, together with the outer sheath (120,220), in the forward direction (to the right, in the figure). This configuration is useful when there is a need to pierce, or shoot through, a tissue portion. As the needle and sheath are moved together, the cavity remains covered during this motion. As shown, the biopsy device includes a movement mechanism **700** similar to the movement mechanism 400 described with reference to Figs. 5A-5K , i.e. the movement of the outer sheath backwards and forwards relative to the inner needle is the same as described above in movement mechanism 400, including the closed route **712,** being the same as route 412 described above. The back side of the inner needle is coupled to a spring **706** via a coupler **708.** When the inner needle is in its backward position, as shown in the figure, the spring **706** is energized, by compression, and held in place by a latch **710.** When the latch **710** is released, the spring **706** starts to relax to the right side thus pushing the coupler **708** forwardly and with it the inner needle and the sheath, while maintaining the relative position between the needle and sheath, i.e. the sampling cavity remains fully covered during this urging motion. This is achieved by pin **716,** which is fixedly attached at its back side to the coupler **708** and is pushed at its front side against the backside of the outer sheath. Manual sliding of the latch **710** backwardly energizes and engages the spring **706,** readying the device for another urging movement in the forward direction of the inner needle together with the outer sheath. Again, it should be mentioned that the addition of controllably moving forwards the inner needle together with the outer sheath may be implemented in any of the other embodiments presented in Figs. 4, 7, and 8.

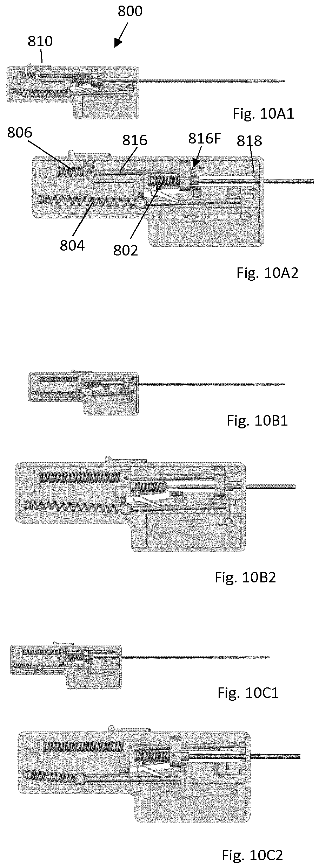

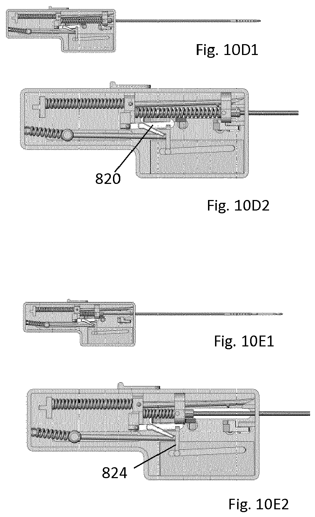

Reference is made to **Figs. 10A-10E** illustrating another non-limiting example of the movement mechanism of the invention. The figures show the different movement stages of the inner needle and the outer sheath. In this example, the movement mechanism **800** combines features of the movement mechanism **700,** as described in Fig. 9, with a unified slanted/inclined route **812A**, being the same as route 312A as described in Figs. 4I-4L. Specifically, **Figs. 10A1 and 10A2** illustrate a situation when the movement mechanism is ready to fire the inner needle and the outer sheath (in the forward position) together forwardly, such that the cavity is kept closed. In this position, the spring **806** which is responsible for the forward firing is compressed. Springs **802** and **804** do not participate in the common forward movement. The latch **810** is activated to release the spring **806** that starts to relax to the right side of the page, pushing with it the inner needle and the outer sheath. The outer sheath is pushed forwardly by the pin **816** which engages at its front side **816F** with the outer sheath. The end of the common forward movement is illustrated in **Figs. 10B1 and 10B2**. The pin **816** is forced to move upwardly because of the protrusion **818** and as a result it detaches from the outer sheath which now becomes free to move in the backward direction. The backward and forward movement of the outer sheath is the same as described in Figs. 5A-5K with respect to movement mechanism 400. **Figs. 10C1 and 10C2** illustrate the backward movement of the outer sheath by the relaxation of the stretched spring **804.** **Figs. 10D1 and 10D2** illustrate the forward movement of the outer sheath by the relaxation of spring **802** after it has been released from the hinge **820.** **Figs. 10E1 and 10E2** illustrate the retraction and back-locking of the outer sheath by pulling it against the spring **802,** to enable access to the cavity. After pushing the pin **824** to the right side against the spring **804,** and pulling the inner needle backwardly by pulling latch **810** to the left side of the page, another cycle of two-stage movement, forward common firing and outer sheath reciprocal movement, can be carried out.

Thus, the present invention, as described and exemplified in the above-mentioned embodiments, provides a novel biopsy device that enables acquisition of high-quality biopsy samples, by precisely and immediately cutting tissue which has been examined. This is achieved, inter alia, by utilizing a novel placement of a tissue sensing unit above the exact location of the cavity the receives the tissue, by utilizing a novel movement of the biopsy device's covering sheath, such that it reciprocates once, backwardly to reveal the cavity under the sensing unit and forwardly to cut the relevant tissue portion, and/or by utilizing a novel suction system which improves the attachment of the tissue portion to the cavity lumen enabling effective and fast cutting of the tissue.

## Claims

1. A biopsy device comprising:
- an inner needle comprising a tip configured to pierce tissue, and a cavity configured for receiving a tissue portion from a region of interest;
- an outer sheath configured to move with respect to the inner needle between a forward position of the sheath in which it totally covers the cavity and a backward position of the sheath in which the cavity is totally uncovered, the outer sheath comprising a cutting edge at its front end configured to cut said tissue portion into said cavity while moving in a forward direction; and
- a tissue sensing unit for characterizing tissue properties, being mounted on the outer sheath such that the tissue sensing unit is aligned with the cavity when the sheath is in the forward position to thereby sense tissue signals from said tissue portion.

2. The biopsy device of claim 1, wherein said tissue sensing unit has at least one of the following configurations:
a) the tissue sensing unit comprises an array of spaced-apart tissue characterization sensors arranged above and along the cavity when the sheath is in the forward position;
b) the tissue sensing unit comprises near-field electromagnetic sensors;
c) the tissue sensing unit comprises capacitance sensors; and
d) the tissue sensing unit is integral with the sheath.

3. The biopsy device of claim 1 or 2, comprising a movement mechanism connected to said sheath at a backside thereof and configured for controllably moving said sheath, in a continuous manner and in response to a single activation, firstly in a backward direction from said forward position to said backward position, and secondly in said forward direction from said backward position to said forward position.

4. The biopsy device of claim 3, comprising a handle being connected at the backside of the outer sheath and accommodating said movement mechanism.

5. The biopsy device of claim 3 or 4, wherein said movement mechanism comprises a locking mechanism comprising a slider and a latch configured respectively to enable retraction and fixation of said sheath into said backward position, to thereby enable extraction of said tissue portion from said cavity.

6. The biopsy device of any one of claims 3 to 5, wherein said movement mechanism comprises a spring which is manually energized prior to activating movement of said sheath.

7. The biopsy device of any one of claims 3 to 6, wherein said movement mechanism has one of the following configurations:
a) the movement mechanism comprises at least first and second springs, said first spring is energized prior to activating movement of said sheath to thereby move said sheath in the backward direction to said backward position, said second spring is energized by relaxation movement of said first spring to thereby move said sheath in the forward direction, from said backward position to said forward position;
b) the movement mechanism comprises at least first and second springs both of which are energized prior to activating movement of said sheath, relaxation movement of said first spring causes movement of said sheath in the backward direction to said backward position, followed by disengagement of said second spring which relaxation movement causes movement of said sheath in the forward direction, from said backward position to said forward position;
c) the movement mechanism comprises a Scotch yoke mechanism comprising a torsion spring configured to be energized and to attach during relaxation to a rotating disk of the scotch yoke mechanism, relaxation movement of said energized torsion spring causes movement of said sheath in the backward and forward directions; or
d) the movement mechanism comprises a torsion spring configured to be energized and a piston engaged at one side with the torsion spring and at a second side with said outer sheath, relaxation movement of said energized torsion spring causes movement of said sheath in the backward and forward directions via said piston.

8. The biopsy device of any one of claims 3 to 7, wherein said movement mechanism is further configured to move said outer sheath and inner needle together forwardly while keeping the cavity covered by the outer sheath.

9. The biopsy device of claim 8, wherein said movement mechanism comprises a spring and a pin, relaxation of the spring causes said outer sheath and inner needle to move forwardly, said pin is attached at one point to said inner needle and engages at another point, during movement, with the outer sheath, to thereby prevent relative movement between the outer sheath and inner needle while moving together forwardly.

10. The biopsy device of any one of the preceding claims, further comprising a suction system configured to apply suction force in said cavity to thereby pull said tissue portion into said cavity while the sheath is moving in the backward direction.

11. A biopsy device comprising:
- an inner needle comprising a tip configured to pierce tissue, and a cavity configured for receiving a tissue portion from a region of interest;
- an outer sheath configured to move with respect to the inner needle between a forward position of the sheath in which it totally covers the cavity and a backward position of the sheath in which the cavity is totally uncovered, the outer sheath comprising a cutting edge at its front configured to cut said tissue portion into said cavity while moving in a forward direction; and
- a suction system configured to apply suction force in said cavity to pull said tissue portion into said cavity while the sheath is moving in a backward direction.

12. The biopsy device of claim 11, comprising a movement mechanism connected to said sheath at a backside thereof and configured for controllably moving said sheath, in a continuous manner and in response to a single activation, firstly in a backward direction from said forward position to said backward position, and secondly in said forward direction from said backward position to said forward position.

13. The biopsy device of claim 11 or 12, wherein said movement mechanism comprises a locking mechanism comprising a slider and a latch configured respectively to enable retraction and fixation of said sheath into said backward position, to thereby enable extraction of said tissue portion from said cavity.

14. The biopsy device of any one of claims 11 to 13, wherein said movement mechanism has one of the following configurations:
a) the movement mechanism comprises at least first and second springs, said first spring is energized prior to activating movement of said sheath to thereby move said sheath in the backward direction to said backward position, said second spring is energized by relaxation movement of said first spring to thereby move said sheath in the forward direction, from said backward position to said forward position;
b) the movement mechanism comprises at least first and second springs both of which are energized prior to activating movement of said sheath, relaxation movement of said first spring causes movement of said sheath in the backward direction to said backward position, followed by disengagement of said second spring which relaxation movement causes movement of said sheath in the forward direction, from said backward position to said forward position;
c) the movement mechanism comprises a Scotch yoke mechanism comprising a torsion spring configured to be energized and to attach during relaxation to a rotating disk of the scotch yoke mechanism, relaxation movement of said energized torsion spring causes movement of said sheath in the backward and forward directions; or
d) the movement mechanism comprises a torsion spring configured to be energized and a piston engaged at one side with the torsion spring and at a second side with said outer sheath, relaxation movement of said energized torsion spring causes movement of said sheath in the backward and forward directions via said piston.

15. A system for use in biopsy procedure, comprising:
a biopsy device, comprising:
an inner needle comprising a tip configured to pierce tissue, and a cavity configured for receiving a tissue portion from a region of interest;
an outer sheath configured to move with respect to the inner needle between a forward position of the sheath in which it totally covers the cavity and a backward position of the sheath in which the cavity is totally uncovered, the outer sheath comprising a cutting edge at its front end configured to cut said tissue portion into said cavity while moving in a forward direction; and
a tissue sensing unit for characterizing tissue properties, being mounted on the outer sheath such that the tissue sensing unit is aligned with the cavity when the sheath is in the forward position to thereby sense tissue signals from said tissue portion; and
a control unit connected to said tissue sensing unit, being configured and operable for:
sending signals to said tissue sensing unit;
receiving signals therefrom when said outer sheath is in the forward position; and
generating data indicative of a condition of the tissue portion, to thereby enable identifying locations inside the tissue at which tissue samples are to be cut and collected in said cavity.
